# EUROPEAN PATENT APPLICATION

(11) **EP 2 065 025 A2**
(43) Date of publication of application: **03.06.2009**
(21) Application number: 08020709.5
(22) Date of filing: 28.11.2008
(51) Int. Cl.: A61K 8/37, A61Q 19/00

(54) **A preservative composition and its use, a method of preparing it and formulations containing it**

(30) Priority: 28.11.2007 IT to20070857
(71) Applicant: Capelli, Cristina, 16146 Genova (GE) (IT)
(72) Inventor: Capelli, Cristina, 16146 Genova (GE) (IT)
(74) Representative: Ferreccio, Rinaldo

(57) **Abstract**

A Composition comprising glyceryl caprylate and a glycerine/water/sodium levulinate/sodium anisate mixture.

In particular, the composition comprises: 0.25%-3.0% of glyceryl caprylate, and 0.5%-7.0% of a glycerine/water/sodium levulinate/sodium anisate mixture.

A method for the preparation of the composition, its use as a preservative and a formulation comprising it are also provided.

## Description

The present invention relates to a preservative composition, to the method for preparing it, to its use and to a formulation containing it.

Preservatives are natural or synthetic substances that allow food, pharmaceutical and cosmetic products to be preserved for long periods protecting them from deterioration caused by microorganisms.

Numerous synthetic preservatives are known, used above all in the field of cosmetics, like for example methylchloroisothiazoline, 2-bromo-2-nitropropane-1,3-diol, sodium dehydroacetate, imidazolidinyl urea, phenoxyethanol and parabens.

All of these preservatives have, however, proven to have an irritant if not actually toxic action, particularly if used at high concentrations.

In particular, methylchloroisothiazoline and imidazolidinyl urea, which are present in wet wipes, are known as sensitizers. De Groot et al in 1991 observed that methylchloroisothiazoline (MI/MCI), also known as Kathon CG developed allergic dermatitis in 5 patients (De Groot et al., Contact. Dermatitis 1991; 24:135-136). The symptoms improved after use of the wet wipes was stopped.

It is also known that parabens, preservatives which are widely used in the cosmetics industry, can produce intolerances, above all for particularly sensitive skin.

2-bromo-2-nitropropane-1,3-diol is also known for having caused cases of contact allergic dermatitis.

Products of natural origin, on the other hand, include Dermosoft 1388®, commercialised by Dr. Straetmans GmbH (DE), comprising a glycerine/water/sodium levulinate/sodium anisate mixture. Disadvantageously, however, such a mixture does not ensure a total preservative action in the end products and, indeed, it is not included in the list of preservatives permitted for cosmetic use.

Currently, therefore, a preservative agent without the drawbacks of known preservatives is being sought.

The purpose of the present invention is therefore to find a natural preservative that has a preserving activity greater than or at least comparable to that of known synthetic preservatives and that does not cause adverse reactions on the end user once used.

According to the present invention, such a purpose is accomplished through the composition according to claim 1, its uses according to claims 5 and 6, and a method for preparing it according to claim 7.

A formulation according to claim 8 is also provided.

The expression "glycerine/water/sodium levulinate/sodium anisate mixture", as used in the present description and in the claims, should be taken to preferably correspond to the product Dermosoft 1388® or to products having a composition substantially corresponding to that of the product Dermosoft 1388®.

Advantageously, it has been demonstrated that glyceryl caprylate, a substance not classified as a preservative but indicated by the International Nomenclature Cosmetic Ingredients as a emollient/emulsifier, when added to a glycerine/water/sodium levulinate/sodium anisate mixture, acts synergically with such a component giving excellent results in terms of preservation.

Preferably, according to the present invention, a composition is provided comprising:
- 0.25-3.0% of glyceryl caprylate, and
- 0.5-7.0% of glycerine/water/sodium levulinate/sodium anisate mixture, and more preferably:

- 0.5-2.0% of glyceryl caprylate, and
- 1.0-5.0% of a glycerine/water/sodium levulinate/sodium anisate mixture.

In a preferred embodiment the composition comprises:
- 1% of glyceryl caprylate, and
- 4% of a glycerine/water/sodium levulinate/sodium anisate mixture.

The composition has proven particularly effective as a preservative especially when used to prepare formulations, preferably cosmetics.

Such formulations can, for example, be:
- Skin creams, emulsions, lotions, gels and oils
- Beauty masks
- Foundations (liquids, pastes, powders)
- Makeup powder, talcum powder for use after bath and for personal hygiene, etc.
- toilet soaps, deodorant soaps, etc.
- Perfumes, eaux de toilette and eaux de Cologne
- Bath and shower preparations (salts, foams, oils, gels, etc.)
- Hair-removal products
- Deodorants and antiperspirants
- Hair treatment products
- Hair dyes and bleaches
- Products for curling, straightening and fixing hair
- Hair-setting products
- Hair-cleaning products (lotions, powders, shampoos)
- Products for keeping hair in position (lotions, cream, oils)
- Hair-styling products (lotions, sprays, brillantines)
- Shaving products (soaps, foams, lotions, etc.)
- Make-up and make-up removing products for the face and the eyes,
- Products intended to be applied onto the lips
- dental and oral hygiene products.
- Nail hygiene products and nail varnishes
- External intimate hygiene products
- Suncare products
- Artificial tanning products
- Skin whitening products
- Anti-wrinkle products

The composition is prepared by heating a glycerine/water/sodium levulinate/sodium anisate mixture to a temperature not higher than 40°C and then adding glyceryl caprylate under continuous and slow stirring until the solution thus obtained becomes totally clear.

Further features of the present invention shall become clear from the following description of some examples given merely for illustrative and not limiting purposes.

### Example 1

The preservative composition according to the invention is prepared by heating one part of a glycerine/water/sodium levulinate/sodium anisate mixture to a temperature of 40°C. Then four parts of glyceryl caprylate are added under continuous and slow agitation until totally clear.

The composition thus obtained is added to a moisturising cream after emulsification and at a temperature below 40°.

### Example 2

The moisturising cream prepared according to example 1 is microbiologically tested to determine the total bacteria, yeast, fungus and mould count immediately after preparation and after 4-month storage.

A series of dilutions (1:10,1:100 and 1:1000) of the moisturising cream is prepared in universal dilutant comprising soy lecithin, Polysorbate 80, sodium thiosulfate pentahydrate, L-Histidine hydrochloride, Triptone, sodium chloride, disodium phosphate 12 H₂O, monopotassium phosphate and distilled water.

The culture media used are PCA (Plate Count Agar) to search for bacteria and YDC (Yeast Extract Dextrose calcium carbonate agar) to search for yeast and mould.

From every dilution 1 ml is taken and distributed in Petri dishes; then, for each dilution a plate to which 15-20 ml of PCA are added and a plate to which 15-20 ml of YDC are added are prepared. After suitable mixing and solidification the plates are placed in incubation for 72 hours at 30°C (plates containing PCA) or for 5 days at 24°C (plates containing YDC).

The data obtained is shown in Table I.

**Table I**

| | Bacteria count | Mould and yeast count |
|---|---|---|
| After preparation | 20 ufc/ml | < 10 ufc/ml |
| After storage for 4 months | 10 ufc/ml | < 10 ufc/ml |

From the results obtained it can be seen that there is a regression in the microbe count that indicates not only the good preserving activity of the composition according to the invention, but also a microbicidal capability thereof.

### Example 3

With the same method illustrated in example 1 a shampoo was prepared comprising the preservative composition according to the present invention.

Such a shampoo was subjected to a Challenge test to determine the preserving power of the mixture.

Initially, it was checked that the sample of shampoo did not have a bacterial charge.

Then the sample was inoculated with different strains: (A)Staphylococcus aureus 2.1 x 10⁷, (B) Pseudomonas aeruginosa 2.1 x 10⁷, (C)Escherichia coli 2.1 x 10⁷, (D)Candida albicans 2.1 x 10⁷, (E)Aspergillus niger 2.1 x 10⁷.

After 7 days the results illustrated in Table II were obtained.

**Table II**

| | Colony- forming units/g after inoculation | | | | |
|---|---|---|---|---|---|
| time | (A) | (B) | (C) | (D) | (E) |
| 7 days | <10 | <10 | <10 | <10 | <10 |

The results illustrated in Table II were compared with those of a shampoo containing Dermosoft 1388 as preservative composition, subjected to the same Challenge Test procedure.

The data obtained is shown in Table III.

**Table III**

| | Colony-forming units/g after inoculation | | | | |
|---|---|---|---|---|---|
| time | (A) | (B) | (C) | (D) | (E) |
| 7 days | <10 | <10 | <10 | 20 | 3.2x10² |

The data shown above illustrates how the preservative composition according to the invention, in which a glycerine/water/sodium levulinate/sodium anisate mixture is added to glyceryl caprylate, has a greater preservative power with particular reference to fungi and yeast than the glycerine/water/sodium levulinate/sodium anisate mixture by itself.

## Claims

1. A composition comprising glyceryl caprylate and a glycerine / water/ sodium levulinate / sodium anisate mixture.

2. A composition according to claim 1 **characterised in that** it comprises:
- 0.25-3.0% of glyceryl caprylate, and
- 0.5-7.0% of a glycerine/water/sodium levulinate/sodium anisate mixture.

3. A composition according to claims 1 or 2, **characterised in that** it comprises:
- 0.5-2.0% of glyceryl caprylate, and
- 1.0-5.0% of a glycerine/water/sodium levulinate/sodium anisate mixture.

4. A composition according to any one of the previous claims, **characterised in that** it comprises:
- 1% of glyceryl caprylate,
- 4% of a glycerine/water/sodium levulinate/sodium anisate mixture.

5. Use of a composition according to any one of claims 1 to 4 as a preservative.

6. Use of a composition according to any one of claims 1 to 4 for the preparation of a cosmetic formulation.

7. A method for the preparation of the composition according to any one of claims 1 to 4 **characterised in that** it comprises the steps of heating a glycerine/water/sodium levulinate/sodium anisate mixture to a temperature not higher than 40°C and adding glyceryl caprylate under continuous and slow agitation until totally clear.

8. A formulation comprising a composition according to any one of claims 1 to 4.
